# EUROPÄISCHE PATENTSCHRIFT

(11) **EP 0 326 135 B1**
(45) Veröffentlichungstag und Bekanntmachung des Hinweises auf die Patenterteilung: **31.03.1993**
(21) Anmeldenummer: 89101354.2
(22) Anmeldetag: 26.01.1989
(51) Int. Cl.: G01N 33/545, D21H 17/34

(54) **Trägervlies für ablösbar imprägnierte Reagenzien**
Carrier fleece for impregnated releasable reagents
Toison de support pour des réactifs imprégnés détachables

(30) Priorität: 27.01.1988 DE 3802366
(43) Veröffentlichungstag der Anmeldung: 02.08.1989
(73) Patentinhaber: BOEHRINGER MANNHEIM GMBH, 68298 Mannheim (DE)
(72) Erfinder: Baier, Manfred, Dr., D-8124 Seeshaupt (DE); Jering, Helmut, Dr., D-8132 Tutzing (DE); Lerch, Rolf, Dipl.-Ing., D-6804 Ilvesheim (DE); Mangold, Dieter, Dr., D-6701 Maxdorf (DE); Mössner, Ellen, Dr., D-8132 Tutzing (DE); Pappert, Gunter, Dr., D-8132 Tutzing (DE); Nötzel, Siegfried, Dr., D-6901 Wilhelmsfeld (DE)
(74) Vertreter: Huber, Bernhard, Dipl.-Chem.

(56) Entgegenhaltungen:
- EP-A- 0 226 182
- EP-A- 0 267 519
- EP-B- 167 171
- EP-B- 167 175
- DE-A- 2 949 305
- DE-B- 2 158 124
- US-A- 4 515 889
- US-A- 4 588 555
- ABSTRACT BULLETIN, Band 58, Nr. 3, September 1987, Appleton, WI (US); Seite 410, Nr. 3676*
- ABSTRACT BULLETIN, Band 58, Nr. 1, Juli 1987, Appleton, WI (US); Seite 137, Nr. 1069*
- ABSTRACT BULLETIN, Band 57, Nr. 11, Mai 1987, Appleton, WI (US); Seite 1602, Nr. 14601*

## Beschreibung

Die Erfindung betrifft ein Trägervlies für ablösbar imprägnierte Reagenzien.

In der klinischen Diagnostik sowie auch in der Analyse von Lebensmitteln, Bedarfsgegenständen und Wasser werden viele Parameter sehr häufig bestimmt. Dazu werden oft Nachweisverfahren unter Verwendung von Enzymen oder Nachweisverfahren nach dem Prinzip des Immunoassays unter Verwendung immunologisch aktiver Substanzen durchgeführt. Für diese ständig durchzuführenden Bestimmungen sind schon vorbereitete Testkits im Handel, die alle für eine Analyse erforderlichen Komponenten enthalten. In der einfachsten Form liegen dabei die einzelnen Komponenten als Lösungen vor, die dann leicht und schnell zu dosieren sind. Insbesondere bei Verwendung von Analyseautomaten, die zunehmend in großen Labors für die Routinediagnostik verwendet werden, sind Bestimmungsverfahren unter Verwendung von Reagenzien in Lösungen am einfachsten durchzuführen. Viele Substanzen, insbesondere biologisch aktive Moleküle, sind jedoch in Lösung nicht stabil und können daher in dieser Form nicht über einen längeren Zeitraum aufbewahrt werden. Diese Substanzen werden daher entweder in Form von Tabletten eingesetzt (Siehe z.B. US-A-4 515 889, Spalte 4, Zeile 13), bei denen sich jedoch Probleme ergeben, da sie entweder zu hart gepreßt sind und sich dann schwer auflösen oder aber eine ungenügende Härte aufweisen und daher abbröckeln, so daS die Dosierung ungenau wird. Weiterhin ist es bekannt, Lyophilisate zu verwenden (Siehe z.B. US-A-4 588 555, Spalte 4, Zeile 4), die bei Verwendung für den Test durch Zugabe eines Lösungsmittels, das meistens Wasser ist, rekonstituiert werden. Nachteil der Lyophilisate ist jedoch das Verfahren ihrer Herstellung, das sehr aufwendig und teuer ist.

Um diese Nachteile zu vermeiden, wurde bereits vorgeschlagen, Papiervliese mit Reagenzien zu imprägnieren und dann diese Papiervliese in die Reaktionslösung während des Bestimmungsverfahrens einzubringen (Siehe z.B. EP-B-0 167 171 und EP-B-0 167 175). Dazu muß das Reagenz einerseits auf dem Papiervlies sehr gut haften, damit nicht durch vorzeitige Ablösung die Menge, die auf dem Vlies imprägniert ist, verändert wird. Andererseits ist es notwendig, daß bei Einbringen in die Reaktionslösung das aufgebrachte Reagenz schnell und vollständig eluiert wird. Die bisher bekannten Papiervliese befriedigen noch nicht, da sie entweder das aufgebrachte Reagenz zu wenig binden, so daß schon während der Lagerung ein Teil des aufgebrachten Reagenzes sich ablöst oder aber die Bindung des Reagenzes ist so stark, daß es nicht schnell und vollständig eluiert werden kann.

Für biologisch aktive Materialien stellt sich weiterhin das Problem der Stabilität. Insbesondere Enzyme und immunologisch aktive Substanzen verlieren bei längerer Lagerung ihre Aktivität, was sich auf die Nachweisverfahren, in denen sie verwendet werden, nachteilig auswirkt.

Es war nun Aufgabe der Erfindung, ein Trägervlies zu schaffen, mit dem Reagenzien, insbesondere Konjugate von Haptenen, Antigenen und Antikörpern mit Markierungssubstanzen und Proteinkonjugaten ablösbar imprägniert werden können. Diese Trägervliese sollen die Reagenzien so binden, daß kein Aktivitätsverlust auftritt. Es dürfen weder Enzyme die allein oder als Konjugate imprägniert werden, noch biologisch aktive Substanzen, ihre Aktivität durch Trocknung und Lagerung verlieren. Das Konjugat soll bei Eintauchen in die Probelösung leicht und vollständig eluiert werden können. Weiterhin ist es wesentlich, daß das Trägervlies pro Flächeneinheit eine definierte reproduzierbare Menge des Reagenzes aufnimmt. Weiterhin muß das Trägervlies so beschaffen sein, daß es sich maschinell zerteilen läßt sowohl in nassem als auch in trockenem Zustand. Es darf weder im nassen, noch im trockenen Zustand reißen. Außerdem soll das Trägervlies das Reagenz so stabilisieren, daß auch bei langer Lagerung kein Aktivitätsverlust auftritt.

Diese Aufgabe wird nun gelöst durch ein Trägervlies für ablösbar imprägnierte Reagenzien, das dadurch gekennzeichnet ist, daß es aus
a) Fasern auf Basis von Cellulose,
b) Polymerisatfasern auf Basis von Polyester und/oder Polyamid und
c) einem organischen Bindemittel, das OH- und/oder Estergruppen aufweist,
zusammengesetzt ist.

Überraschenderweise wurde festgestellt, daß ein Vlies, das die obengenannte Zusammensetzung aufweist, alle genannten Aufgaben erfüllt. Es führt zu einer Stabilisierung der Reagenzien, so daß auch nach sehr langer Lagerung kein Aktivitätsverlust auftritt. Darüberhinaus läßt sich das Trägervlies maschinell sehr gut zerteilen, reißt aber weder im feuchten, noch im trockenen Zustand. Das aufgebrachte Reagenz wird bei der Lagerung nicht abgelöst, wird aber bei Eintauchen in die Probelösung sofort und vollständig eluiert. Eine unspezifische Bindung von in der Probelösung vorliegenden Substanzen tritt nicht auf.

Um diese vorteilhaften Eigenschaften zu erreichen, muß das Trägervlies aus drei Komponenten bestehen. Die erste Komponente sind Fasern auf Basis von Cellulose. Heer eignen sich alle bekannten Fasern, die überwiegend aus Cellulose bestehen. Bevorzugt werden als Cellulosefasern eingesetzt Zellwolle, Zellstoff und Linters. Als Zellwolle wird ein Material bezeichnet, das durch Alkalisieren von Cellulose zu Alkalicellulose, anschließende Behandlung mit Schwefelkohlenstoff unter Bildung von Cellulose-Xanthogenaten, Auflösung der Cellulose-Xanthogenate in Lauge und Verspinnen zu Viskosefilamentgarn erhalten wurde. Zellstoff kann durch einen vollständigen chemischen Aufschluß cellulosehaltiger Materialien und anschließender Bleiche gewonnen werden. Als Linters werden kurze, nicht spinnbare Baumwollfasern bezeichnet, daß aus Baumwollsamen erhalten werden.

In einer bevorzugten Ausführungsform des erfindungsgemäßen Trägervlieses wird eine Mischung aus Zellwolle und Linters bevorzugt im Verhältnis 5 bis 1:1 verwendet.

Die zweite Komponente sind Polymerisatfasern auf Basis von Polyester und/oder Polyamid. Dabei können reine Polyester und/oder Polyamidfasern sowie Mischfasern verwendet werden.
Besonders bevorzugt werden als Polyamide mit einem spezifischen Gewicht von 1,14 bis 1,15 g/cm³, einer Schnittlänge von 4 - 6 mm und einer Faserfeinheit von 2,2 dtex verwendet.
Besonders bevorzugte Polyesterfasern sind Fasern mit einem spezifischen Gewicht von ca. 1,17 g/cm³, einer Schnittlänge von 3 - 6 mm und einer Faserfeinheit von 1,7 - 3,3 dtex.

Die Cellulose- und Polymerisatfasern weisen bevorzugt eine Faserfeinheit von 1,7 bis 4,5 Detex auf und besitzen Längen von 3 bis 12 mm.

Als weiteren wesentlichen Bestandteil enthält das erfindungsgemäße Trägervlies ein organisches Bindemittel, das OH- und/oder Estergruppen aufweist. Bevorzugt werden hierzu Polyvinylalkohol und Acrylsäureester eingesetzt (Polyacrylsäureester).

Der Polyvinylalkohol wird vorzugsweise als Fasermaterial mit einer Schnittlänge von 4 mm und einem spezifischen Gewicht von 1,26 - 1,30 g/cm³ eingesetzt. Der Acrylsäureester ist eine nichtionogene, selbstvernetzende Acrylharz-Dispersion.

Aus diesen drei Komponenten und Wasser wird ein Trägervlies auf einer Schrägsiebmaschine nach den üblichen Verfahren der Papierherstellung erzeugt. Es sind keine weiteren Zusätze und Hilfsstoffe erforderlich.

Die drei erfindungswesentlichen Komponenten können in weiten Grenzen variieren. Bevorzugt setzt sich das erfindungsgemäße Trägervlies aus 5 bis 60 Gew.-% cellulosehaltigen Fasern und 40 bis 95 Gew.-% Polymerisatfasern zusammen und enthält, bezogen auf das Gewicht des Faseranteils, 5 bis 30 Gew.-% Bindemittel. Werden als Polymerisatfasern Polyesterfasern verwendet, so liegt der Anteil der Polymerisatfasern bevorzugt im oberen Bereich, d.h. bei 60 bis 95%. Werden als Polymerisatfasern Polyamidfasern verwendet, so liegt der Anteil bevorzugt im unteren Bereich, insbesondere bei 40 bis 60%.

Das erfindungsgemäße Trägervlies ist sehr gut zur Imprägnierung mit Reagenzien für immunologische Bestimmungen geeignet. Ein weiterer Gegenstand der Erfindung ist daher die Verwendung des erfindungsgemäßen Trägervlieses zur ablösbaren Imprägnierung von Reagenzien für immunologische Bestimmungen wie z.B. von Haptenen, Antigenen, Antikörpern und/oder deren Fragmenten sowie Konjugaten dieser immunologisch aktiven Substanzen mit Markierungssubstanzen sowie von synthetischen Peptiden. Ebenso geeignet ist das erfindungsgemäße Trägervlies zur ablösbaren Imprägnierung von sonstigen Bindungspartnern wie z.B. Biotin/Avidin/Streptavidin, Protein A/IgG oder Concanavalin A/Mannose.

Bevorzugt werden die erfindungsgemäßen Trägervliese zur ablösbaren Imprägnierung von Konjugaten aus immunologisch aktiven Substanzen und Markierungssubstanzen, wie z.B. Enzyme verwendet. Besonders bevorzugt werden sie zur ablösbaren Imprägnierung von β-Galactosidase-Konjugaten eingesetzt.

Überraschenderweise hat sich gezeigt, daß mit den erfindungsgemäßen Trägervliesen eine ausgezeichnete Stabilität der imprägnierten Reagenzien erreicht werden kann. Dies ist insbesondere deswegen überraschend, da die einzelnen Komponenten des erfindungsgemäßen Trägervlieses diese Stabilisierung nicht bewirken können.

Die Erfindung wird durch die folgenden Beispiele erläutert:

### Beispiel 1

Es wurde ein Trägervlies hergestellt, das aus 80 Teilen Polyesterfasern einer Faserfeinheit von 3,3 dtex und einer Faserlänge von 4 mm besteht, 20 Teilen Zellwolle mit einer Faserfeinheit von 1,7 dtex und einer Schnittlänge von 3 mm, sowie 20 Teilen Polyvinylalkoholfasern einer Schnittlänge von 4 mm. Die Faserstoffe Polyester, Zellwolle und Polyvinylalkohol wurden mit Wasser bei einer Stoffdichte von 0,3 % in Mischbütten aufgeschlagen bzw. vereinzelt.

Der Faserstoff wurde anschließend auf ein umlaufendes Sieb gepumpt. Während das Fasergemisch entwässert bzw. das Wasser durch Vakuum abgesaugt wird, orientieren sich die Fasern auf der Sieboberseite und werden als Vlies mit einem Trockengehalt von ca. 20 % über Trockenzylinder kontaktgetrocknet.

### Beispiel 2

Wie im Beispiel 1 beschrieben, wurde ein Trägervlies hergestellt, das die folgende Zusammensetzung hatte:

| | | |
|---|---|---|
| Polyamid: | Faserfeinheit 2,2 dtex/Schnittlänge 6 mm | 40 % |
| Zellwolle: | Faserfeinheit 1,7 dtex/Schnittlänge 3 mm | 30 % |
| Linters: | | 20 % |
| Polyvinylalkohol: | Schnittlänge 4 mm | 10 %. |

### Beispiel 3

Es wurden verschiedene Trägervliese hergestellt und mit einem Konjugat von polyklonalen Schaf-Antidigoxin-Antikörper-Fab-Fragmenten und β-Galactosidase (PAK<Dig>-S-Fab(IS)-β-Gal) imprägniert. Die Trägervliese wurden so imprägniert, daß jedes Trägervlies eine Enzymaktivität von 1249 mE aufwies. Diese Trägervliese wurden in eine wäßrige Lösung gegeben und die in die Lösung übergegangene Aktivität bestimmt. Die entsprechende Konjugatmenge wurde in 20 µl 100 mmol/l Hepes, pH 7,25 + 1,5 % Polyethylenglycol (PEG 6000) gelöst und ein Vlies (6x8,4 mm) mit dieser Lösung getränkt. Dazu wurde das Vlies mit Metallnadeln aufgespiest und sofort nach der Tränkung 30 Minuten bei 35°C im Umlufttrockenschrank getrocknet, anschließend sofort in Trockenröhrchen gegeben und bei 4°C über Nacht gelagert. Die so imprägnierten Trägervliese wiesen jeweils eine Enzymaktivität von 1249 mE auf. Zur Bestimmung der Eluierbarkeit werden die Vliese mit 1 ml Inkubationspuffer (100 mmol/l Hepes, pH 7,25, 0,9 % NaCl, 2 mmol/l MgAspartat, 0,1 % Rinderserumalbumin, 0,2 % Tween® 20 (Polyoxyethylensorbitancarbonsäureester)) eluiert. Danach wird die in die Lösung übergegangene Enzymaktivität bestimmt. Die Auswertung erfolgt über eine Eichkurve. Die Zusammensetzung der einzelnen Vliese sowie die Ergebnisse sind der folgenden Aufstellung zu entnehmen.

| Vliestyp | eingesetzte Aktivität (in mE) | eluierte Aktivität (in mE) | unspezifische Bindung (in %) |
|---|---|---|---|
| 40 Teile Polyamid | 1249 | 1151 | 8 |
| 30 Teile Zellwolle | | | |
| 20 Teile Linters | | | |
| 10 Teile Polyvinylalkohol | | | |
| 90 Teile Polyester | 1249 | 1257 | 1 |
| 10 Teile Zellwolle | | | |
| 30 Teile Acrylsäureester | | | |
| 80 Teile Polyester | 1249 | 1237 | 1 |
| 20 Teile Zellwolle | | | |
| 20 Teile Polyvinylalkohol | | | |

### Beispiel 4

Erfindungsgemäße Trägervliese, deren Zusammensetzung der folgenden Aufstellung zu entnehmen ist, wurden mit einer Lösung getränkt, die die folgende Zusammensetzung hatte:
50 ± 0,2 mmol/l Hepes pH 7,25
1 ± 0,05 % Protein C
5 ± 0,2 mmol/l Mg-Aspartat
200 U/l PAK<Dig>-S-Fab(IS)-β₋Gal als Lyophilisat.

Die so getränkten Trägervliese wurden auf ihre Stabilität getestet. Sie wurden 3 Wochen bei 35°C mit 2% Feuchte gelagert. Als Referenz wurden Vliese verwendet, die bei +4°C mit 2% Feuchte im Kühlschrank aufbewahrt wurden sowie unbelastete Papiervliese. Zur Bestimmung der Aktivität wurde die enzymatische Aktivität (EA) nach Elution des Reagenzes durch Zugabe von 5 mmol/l Chlorphenolrot-β-D-Galactosid (hergestellt nach DE 33 45 748) in Wasser bestimmt, in dem die Extinktionszunahme bei 578 nm verfolgt wurde.

Die immunologische Aktivität (IA) wurde ebenfalls bestimmt. Dazu wurde zu dem eluierten immunologischen Reagenz ein auf 1:5 verdünntes Humanserum zugegeben, das eine bestimmte Menge an Digoxin (0 bis 5 ng/ml) enthielt. Es wurde 5 Minuten bei 37°C inkubiert und die Mischung dann auf eine Festphase gegeben, die immobilisiertes Digoxin enthielt. Aus der Festphase wird freies Konjugat gebunden, aus dem Überstand kann dann die enzymatische Aktivität wie oben bestimmt werden. Das Meßsignal ist der Digoxinmenge in der Probe proportional.

Die Ergebnisse sind der folgenden Tabelle zu entnehmen.

| Vliestyp | unmittelbar nach Herstellung % Aktivität | | 4°C/3 Wochen % Aktivität | | 35°C/3 Wochen % Aktivität | |
|---|---|---|---|---|---|---|
| | EA | IA | EA | IA | EA | IA |
| 40 Teile Polyamid | 100 | 100 | 98 | 96 | 91 | 97 |
| 30 Teile Zellwolle | | | | | | |
| 20 Teile Linters | | | | | | |
| 10 Teile Polyvinylalkohol | | | | | | |
| 90 Teile Polyester | 100 | 100 | 99 | 97 | 95 | 94 |
| 10 Teile Zellwolle | | | | | | |
| 30 Teile Acrylsäureester | | | | | | |
| 80 Teile Polyester | 100 | 100 | 97 | 98 | 93 | 95 |
| 20 Teile Zellwolle | | | | | | |
| 20 Teile Polyvinylalkohol | | | | | | |

### Beispiel 5

Es wurde ein Vlies hergestellt aus
90 Teilen Polyester,
10 Teilen Zellwolle und, bezogen auf die Fasern,
30 Teilen Acrylsäureester.

Auf dieses Vlies wurde ein Konjugat aufgebracht. Es wurde die Eluierbarkeit geprüft. 99% der aufgebrachten Aktivität befanden sich nach der Elution in der Lösung.

### Beispiel 6

Es wurde ein Vlies hergestellt aus
30 Teilen Polyamid,
20 Teilen Linters,
30 Teilen Zellwolle und
10 Teilen Polyvinylalkohol.

Dieses Vlies wurde mit einer, ein immunologisches Konjugat enthaltenden Lösung getränkt und anschließend getrocknet. Es wurde dann die Eluierbarkeit getestet. Von der aufgebrachten Aktivitätsmenge konnten 98% in der eluierten Lösung nachgewiesen werden.

## Patentansprüche

1. Trägervlies, bestehend aus
(a) Fasern auf der Basis von Cellulose,
(b) Polymerisatfasern auf Basis von Polyester und/oder Polyamid und
(c) einem organischen Bindemittel, das OH- und/oder Estergruppen aufweist,
welches darauf imprägniert Reagenzien für immunologische Bestimmungen gegebenenfalls als Konjugat mit Markierungssubstanzen und/oder synthetische Peptide in ablösbarer Form aufweist.

2. Trägervlies nach Anspruch 1, **dadurch gekennzeichnet,** daß es aus 5 bis 60 Gew.-% Fasern auf Basis von Cellulose, 40 bis 95 Gew.-% Polymerisatfasern und 5 bis 30 Gew.-%, bezogen auf das Gewicht der Fasern, eines organischen Bindemittels zusammengesetzt ist.

3. Trägervlies nach einem der vorhergehenden Ansprüche, **dadurch gekennzeichnet,** daß es aus 60 bis 40 Gew.-% Fasern auf Basis Cellulose, 40 bis 60 Gew.-% Polyamid und 5 bis 30 Gew.-%, bezogen auf das Gewicht der Fasern, Polyvinylalkohol besteht.

4. Trägervlies nach einem der vorhergehenden Ansprüche, **dadurch gekennzeichnet,** daß es aus 5 bis 40 Gew.-% Fasern auf Basis Cellulose, 95 bis 60 Gew.-% Polyester und 5 bis 30 Gew.-%, bezogen auf das Gewicht der Fasern, Polyvinylalkohol besteht.

5. Trägervlies nach Anspruch 1 bis 4, **dadurch gekennzeichnet,** daß die Fasern auf Basis von Cellulose Fasern aus Zellwolle, Linters und/oder Zellstoff sind.

6. Trägervlies nach Anspruch 5, **dadurch gekennzeichnet,** daß die Fasern auf der Basis von Cellulose eine Mischung von Zellwolle und Linters sind.

7. Trägervlies nach einem der vorhergehenden Ansprüche, **dadurch gekennzeichnet,** daß die Polymerisatfasern Polyesterfasern sind.

8. Trägervlies nach einem der vorhergehenden Ansprüche, **dadurch gekennzeichnet,** daß das organische Bindemittel Polyvinylalkohol und/oder Acrylsäureester ist.

9. Trägervlies nach einem der vorhergehenden Ansprüche, **dadurch gekennzeichnet,** daß die darauf imprägnierte Substanz ein Hapten, Antigen, Antikörper oder Fragment davon ist.

10. Trägervlies nach einem der vorhergehenden Ansprüche, **dadurch gekennzeichnet,** daß es β-Galactosidase-Konjugate in ablösbarer Form aufweist.

11. Verwendung eines Trägervlieses nach einem der vorhergehenden Ansprüche für immunologische Bestimmungsverfahren.

## Claims

1. Carrier fleece composed of
a) fibres based on cellulose,
b) polymer fibres based on polyester and/or polyamide and
c) an organic binding agent which contains OH and/or ester groups,
which has, in releasable form, reagents impregnated thereon for immunological determinations, possibly as conjugate with labelling substances and/or synthetic peptides.

2. Carrier fleece according to claim 1, characterised in that it is composed of 5 to 60 wt.% of fibres based on cellulose, 40 to 95 wt.% of polymer fibres and 5 to 30 wt.%, referred to the weight of the fibres, of an organic binding agent.

3. Carrier fleece according to one of the preceding claims, characterised in that it consists of 60 to 40 wt.% of fibres based on cellulose, 40 to 60 wt.% of polyamide and 5 to 30 wt.%, referred to the weight of the fibres, of polyvinyl alcohol.

4. Carrier fleece according to one of the preceding claims, characterised in that it consists of 5 to 40 wt.% of fibres based on cellulose, 95 to 60 wt.% of polyester and 5 to 30 wt.%, referred to the weight of the fibres, of polyvinyl alcohol.

5. Carrier fleece according to claim 1 to 4, characterised in that the fibres based on cellulose are fibres of regenerated cellulose, linters and/or cellulose pulp.

6. Carrier fleece according to claim 5, characterised in that the fibres based on cellulose are a mixture of regenerated cellulose and linters.

7. Carrier fleece according to one of the preceding claims, characterised in that the polymer fibres are polyester fibres.

8. Carrier fleece according to one of the preceding claims, characterised in that the organic binding agent is polyvinyl alcohol and/or acrylic acid ester.

9. Carrier fleece according to one of the preceding claims, characterised in that the substance impregnated thereon is a hapten, antigen, antibody or fragment thereof.

10. Carrier fleece according to one of the preceding claims, characterised in that it has β-galactosidase conjugate in releasable form.

11. Use of a carrier fleece according to one of the preceding claims for immunological determination processes.

## Revendications

1. Nappe de support consistant en
(a) fibres à base de cellulose,
(b) fibres de polymère à base de polyester et/ou de polyamide et
(c) un liant organique qui présente des groupes OH et/ou ester,
sur laquelle sont imprégnés de manière détachable des réactifs pour déterminations immunologiques éventuellement sous forme de conjugués avec des substances de marquage et/ou des peptides synthétiques.

2. Nappe de support selon la revendication 1, caractérisée en ce qu'elle est constituée par 5 à 60% en poids de fibres à base de cellulose, 40 à 95% en poids de fibres de polymère et 5 à 30% en poids, par rapport au poids des fibres, d'un liant organique.

3. Nappe de support selon l'une des revendications précédentes, caractérisée en ce qu'elle consiste en 60 à 40% en poids de fibres à base de cellulose, 40 à 60% en poids de polyamide et 5 à 30% en poids, par rapport au poids des fibres, de poly(alcool vinylique).

4. Nappe de support selon l'une des revendications précédentes, caractérisée en ce qu'elle consiste en 5 à 40% en poids de fibres à base de cellulose, 95 à 60% en poids de polyester et 5 à 30% en poids, par rapport au poids des fibres, de poly(alcool vinylique).

5. Nappe de support selon les revendications 1 à 4, caractérisée en ce que les fibres à base de cellulose sont des fibres de laine de cellulose, de linters et/ou de pâte chimique.

6. Nappe de support selon la revendication 5, caractérisée en ce que les fibres à base de cellulose sont un mélange de laine de cellulose et de linters.

7. Nappe de support selon l'une des revendications précédentes, caractérisée en ce que les fibres de polymère sont des fibres de polyester.

8. Nappe de support selon l'une des revendications précédentes, caractérisée en ce que le liant organique est le poly(alcool vinylique) et/ou un ester de l'acide acrylique.

9. Nappe de support selon l'une des revendications précédentes, caractérisée en ce que la substance imprégnée sur la nappe est un haptène, un antigène, un anticorps ou un fragment de ceux-ci.

10. Nappe de support selon l'une des revendications précédentes, caractérisée en ce qu'elle comporte des conjugués de β-galactosidase sous forme détachable.

11. Utilisation d'une nappe de support selon l'une des revendications précédentes pour des procédés de détermination immunologique.
